# EUROPÄISCHE PATENTSCHRIFT

(11) **EP 2 800 596 B1**
(45) Veröffentlichungstag und Bekanntmachung des Hinweises auf die Patenterteilung: **03.07.2019**
(21) Anmeldenummer: 12829210.9
(22) Anmeldetag: 13.11.2012
(51) Int. Cl.: A61M 5/30, A61M 5/24, A61M 5/315, A61M 5/31, A61M 5/20, B65B 3/00

(54) **ZYLINDER-KOLBEN-EINHEIT MIT MEHRTEILIGEM KOLBEN**
CYLINDER-PISTON UNIT WITH MULTI-PART PISTON
UNITÉ CYLINDRE-PISTON À PISTON EN PLUSIEURS PARTIES

(30) Priorität: 16.11.2011 DE 102011119204
(43) Veröffentlichungstag der Anmeldung: 12.11.2014
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Simandi, Claus
(86) Internationale Anmeldenummer: PCT/EP2012/072506
(87) Internationale Veröffentlichungsnummer: WO 2013/092008

(56) Entgegenhaltungen:
- WO-A1-85/00524
- WO-A1-2007/054233
- FR-A1- 2 805 749

## Beschreibung

Die Erfindung betrifft eine Zylinder-Kolben-Einheit, mit einem, eine Injektionslösung aufnehmenden Zylinder und einem Kolben.

Aus der DE 10 2005 054 600 ist eine Zylinder-Kolben-Einheit eines nadelfreien Injektors bekannt, deren Zylinder und deren Kolben eine Injektionslösung zumindest zeitweise umschließen. Der Zylinder hat an seinem vorderen Ende mindestens eine Austrittsdüse. Der Kolben weist im vorderen Bereich eine elastische Schürze auf, deren vordere Außenkante beim unbelasteten Kolben eine Querschnittsfläche aufspannt, die größer ist als eine Querschnittsfläche der Zylinderinnenwandung.

FR 2 805 749 A1 beschreibt eine gattungsmäßige Zylinder-Kolbeneinheit (Abb. 1), jedoch werden Dichtkörper am Zylinder nicht offenbart. Derartige Kolben lassen sich in der Regel nicht blasenfrei in einen schon befüllten Zylinder einer Zylinder-Kolben-Einheit einsetzen. In der Regel wird dafür eine sogenannte Vakuumsetzmaschine benötigt.

Eine andere Methode, einen Stopfen bei Umgebungsdruck auf den Spiegel der schon eingefüllten Injektionslösung zu setzen, besteht darin, ihn mittels eines Mehrfingergreifers radial komprimiert auf den Flüssigkeitsspiegel aufzusetzen. Dies gelingt aber nur mit elastischen, z.B. aus einer Gummimischung bestehenden Kolben, die in der Regel nicht bis 350^{∗}10⁵ Pa dichten und deshalb für nadelfreie Injektoren ungeeignet sind.

Die US 1 521 890 A beschreibt eine metallische Spritze aus der Medizintechnik, die einen Kolben hat, der ohne ein gummiartiges Dichtmittel auskommt. Dazu hat der Kolben die Form eines nach vorn offenen Topfes, in dessen Boden ein zentrales Innengewinde angeordnet ist. Über das Innengewinde ist der Kolben auf der Kolbenstange so aufgeschraubt, dass letztere auch vorn über den Topf übersteht. Die am Zylinder anliegende Topfwandung verjüngt sich nach vorn hin. Im Hohlraum des Topfes sitzt ein kegelstumpfförmiger Spreizkörper. Er ist ebenfalls auf der Kolbenstange aufgeschraubt. Durch das Einschrauben des Spreizkörpers wird die Topfwandung gegen die Zylinderwandung gepresst.

Der vorliegenden Erfindung liegt daher die Problemstellung zugrunde, einen Kolben zu entwickeln, dessen Bestandteile sich bei Umgebungsdruck zumindest teilweise auf den Flüssigkeitsspiegel eines befüllten Zylinders blasenfrei, steril und gasdicht aufsetzen lassen und trotzdem einem hohen Komprimierungsdruck beim regulären Entleeren der Zylinder-Kolben-Einheit ohne Leckage standhalten.

Diese Problemstellung wird mit den Merkmalen des Hauptanspruchs erfindungsgemäß gelöst. Der Kolben besteht aus einem Treibkörper und einem Dichtkörper. Der einzelne Dichtkörper, der auf den Flüssigkeitsspiegel der Injektionslösung aufgesetzt ist, ist mindestens eine aus einem gummi- oder einem silikonartigen Werkstoff gefertigte Scheibe, die unter einer radialen Klemmwirkung an der Innenwandung des Zylinders anliegt. Der Treibkörper ist ein topfförmiger Körper mit einer umlaufenden elastischen Schürze. Zumindest ein Bereich der Schürze liegt unter einer radialen Klemmwirkung an der Innenwandung des Zylinders an. Die vor und hinter dem Treibkörper gelegenen Zylinderraumbereiche kommunizieren über mindestens eine Ausnehmung miteinander. Der Treibkörper ist hinter dem Dichtkörper im Zylinder angeordnet, wobei die Schürze dem Dichtkörper zugewandt ist. Für den bei Betätigung nach vorn verschiebbaren Treibkörper ist der Dichtkörper während der Vorwärtsfahrt nahezu komplett aufnehmbar, wobei der Verbund aus dem Treibkörper und dem Dichtkörper die
Funktion eines Kolbens hat.

Mit der Erfindung wird die Zylinder-Kolben-Einheit eines nadelfreien Injektors offenbart. Der Injektor, der auch ein Einmalinjektor sein kann, lagert neben der Zylinder-Kolben-Einheit in einem Injektorgehäuse eingebauten, auf einen Kolbenbetätigungsstempel wirkenden Antrieb. Als mögliche Antriebe können Federspeicher, Gasantriebe mit offenbaren Gaskartuschen oder pyrotechnische Antriebe verwendet werden. Bekannte Federenergiespeicher nutzen vorgespannte mechanische oder pneumatische Federn oder Federsysteme. Wird als Antrieb ein aus der DE 10 2010010 699 A1 bekannter Federenergiespeicher benutzt, wird zum Vorspannen und Halten dieses Federenergiespeichers der Kolbenbetätigungsstempel über mindestens einen am oder im Injektorgehäuse angeordneten Stützstab oder Zughaken formschlüssig gehalten. Der oder die Stützstäbe bzw. Zughaken werden mittels eines oder mehrerer Auslöseelemente bis zum Gebrauch des Einmalinjektors in ihrer Sperrposition arretiert. Zum Auslösen des Injektors werden der oder die Stützstäbe bzw. Zughaken freigegeben, so dass sich der Kolbenbetätigungsstempel -unter der Wirkung des Federenergiespeichers - zumindest annähernd parallel zur Mittellinie des Einmalinjektors bewegen kann, um die im Zylinder der Zylinder-Kolben-Einheit vorhandene Injektionslösung über mindestens eine Düse auszustoßen.

Durch die Aufteilung des Kolbens in einen Treibkörper und einen elastischeren Dichtkörper wird eine konstruktive Funktionentrennung durchgeführt. Auf der einen Seite hat man den sehr elastischen Dichtkolben, der sich als Scheibe mit einem Mehrfingergreifer radial so zusammendrücken lässt, dass sein Außendurchmesser für den Setzvorgang um mehrere zehntel Millimeter kleiner ist als der Innendurchmesser des ihn aufnehmenden Zylinders. Nach dem Zurückziehen des Mehrfingergreifers sitzt er gasdicht und blasenfrei auf der Injektionslösung. Der Dichtkörper erfüllt somit die Funktion des einfachen gasdichten Verschließens der Injektionslösung auf der, der regulären Zylinderaustrittsöffnung entgegen gesetzten Zylinderseite. Das Gebinde aus
dem Zylinder, dem Schutzgehäuse, dem Dichtstopfen und der umschlossenen Injektionslösung lässt sich problemlos steril herstellen.

Auf der anderen Seite gibt es den in einem nächsten Schritt einzusetzenden Treibkörper. Letzterer, aus einem formsteifen, ggf. selbstschmierenden Kunststoff hergestellt, wird mit seiner Schürze voraus hinter den Dichtkörper gesetzt. Seine elastische Schürze liegt dicht und rutschfest an der Zylinderinnenwandung an. Beim Auslösevorgang des Injektors wird der Treibkörper schlagartig vom Kolbenbetätigungsstempel gegen den auf dem Flüssigkeitsspiegel aufsitzenden Dichtkörper zubewegt. Der nach vorn geschobene Treibkörper nimmt den Dichtkörper bei seiner Vorwärtsfahrt nahezu komplett auf. Der elastische Dichtkörper drückt dabei die Schürze, unterstützt vom Flüssigkeitsgegendruck, nach dem Prinzip der Selbsthilfe dichtend gegen die Zylinderinnenwandung. Der Treibkörper hat somit die Funktion eines regulären Kolbens. Er trennt dicht und formsteif im Zylinder die Niederdruckseite von der Hochdruckseite. Im Gegensatz dazu hat der Dichtkörper jetzt nur noch die Funktion, die im Treibkörper vorhandene Entlüftungsbohrung zu verschließen.

Weitere Einzelheiten der Erfindung ergeben sich aus den Unteransprüchen und den nachfolgenden Beschreibungen schematisch dargestellter Ausführungsbeispiele.
- Figur 1 :: Zylinder-Kolben-Einheit mit angeformtem Ausblasrohr und Schutzgehäuse;
- Figur 2:: wie Figur 1 , jedoch ohne Schutzgehäuse (Injektor sitzt schon auf der Haut des Patienten);
- Figur 3:: wie Figur 2, jedoch nach dem Ausstoßen der Injektionslösung (Injektor sitzt noch auf der Haut des Patienten);
- Figur 4:: Treibkörper und Dichtkörper außerhalb des Zylinders;
- Figur 5:: Treibkörper und Dichtkörper aus Figur 4 im Zylinder nach der Montage;
- Figur 6:: wie Figur 4, jedoch hat der Treibkörper einen Zapfen;
- Figur 7:: Treibkörper und Dichtkörper aus Figur 6 im Zylinder nach der Montage.

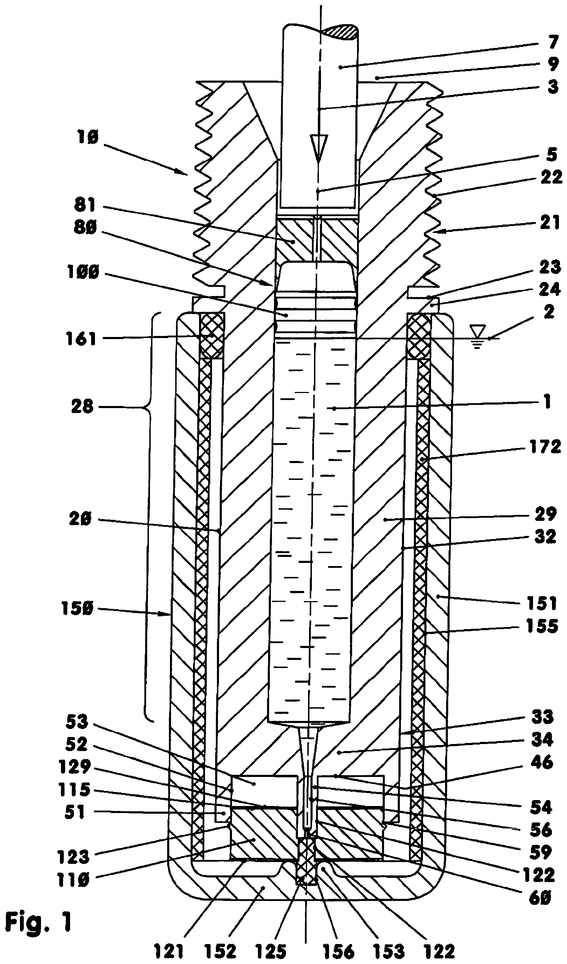

Die Figur 1 zeigt eine Zylinder-Kolben-Einheit (10) eines nadelfreien Injektors. Die Zylinder-Kolben-Einheit (10) besteht aus einem Zylinder (20) und einem zweiteiligen Kolben (80). Der Zylinder (20) ist beispielsweise zusätzlich von einem Schutzgehäuse (150) umgeben. Oberhalb des Kolbens (80) wird der untere Teil eines Kolbenbetätigungsstempels (7) gezeigt, der zu dem hier nicht dargestellten Injektor gehört. Der Zylinder (20) ist mittels seines im hinteren Bereich vorhandenen Außengewindes (22) oder mittels Schlitzen (23) am Injektor befestigt.

Der z.B. einteilige Zylinder (20) besteht aus einem Gehäuseadapter (21), einem Rohrabschnitt (28) und einem Bodenabschnitt (33). Mit dem Gehäuseadapter (21) wird der Zylinder (20) in einem - nicht dargestellten - Injektorgehäuse fixiert. Hierzu weist seine radiale Außenwandung ein Außengewinde (22) und/oder mindestens zwei einander gegenüberliegende Schlitze (23) auf. Die Schlitze (23) haben z.B. eine Tiefe, die der Differenz aus Gewindeaußendurchmesser und Gewindekerndurchmesser entspricht. Sie befinden sich am Gewindeende in unmittelbarer Nähe des Rohrabschnitts (28). Die Breite der Schlitze (23) entspricht z.B. ihrer halben Tiefe.

Zwischen den Schlitzen (23) und dem Rohrabschnitt (28) befindet sich ein Anschlagsteg (24), dessen Außendurchmesser identisch mit dem Gewindeaußendurchmesser ist. Der Außendurchmesser des Rohrabschnittes (28) ist mehr als doppelt so groß wie der Durchmesser der Innenwandung (31). Er ist so dimensioniert, dass sein Werkstoff mindestens einer Druckbelastung von 350 ^{∗}10⁵ Pa standhält.

An den Gehäuseadapter (21) schließt sich die Zylinderwandung (29) des Rohrabschnitts (28) an. Die Zylinderwandung (29) hat entlang der Rohrabschnittslänge z.B. eine konstante Wandstärke von 3,25 mm.

Der Bodenabschnitt (33) umfasst eine nach außen hin z.B. plane Bodenplatte (34), die der mittleren Wandstärke der Zylinderwandung (29) im Bereich des Rohrabschnitts (28) entspricht. Im äußeren Bereich der Bodenplatte (34) ist ein z.B. zylinderrohrförmiger Ringsteg (51) angeformt. Der Ringsteg (51), der einen Klebescheibenaufnahmeraum (53) umspannt, ist beispielsweise so hoch wie die Wandstärke der Bodenplatte (34). Die Wandstärke des Ringstegs (51) beträgt ca. ein Drittel der Wandstärke der Zylinderwandung (29) des Rohrabschnitts (28).

Im Zentrum der planen Bodenplatte (34) ist das die Austrittsdüse (60) tragende Ausblasrohr (54) angeordnet. Das Ausblasrohr (54), dessen Außendurchmesser z.B. 2,25 mm beträgt, hat ein vorderes Ende, das ca. einen Millimeter über den Ringsteg (51) übersteht. Zwischen der zumindest annähernd zylindrischen Außenwandung des Ausblasrohres (54) und der zylindrischen Innenwandung (52) des Ringstegs (51) liegt ein z.B. 3 mm tiefer Klebescheibenaufnahmeraum (53).

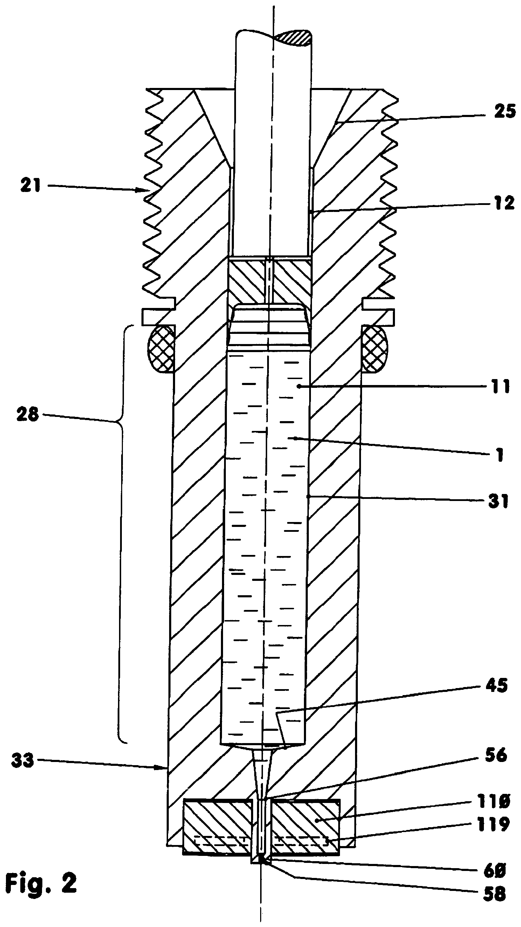

Die Stirnseite (58) des Ausblasrohres (54), vgl. Figur 2, ist zumindest bereichsweise sphärisch gekrümmt oder plan ausgeführt. Die vorn liegende Austrittsdüse (60) hat einen Innendurchmesser von 0,2 bis 0,4 mm, während der Innendurchmesser der zur Austrittsdüse (60) führenden Bohrung (56) zumindest in ihrer vorderen Hälfte ca. 0,5 bis 1 mm misst.

Gemäß Figur 2 ist die Zylinderinnenwandung (31) zumindest im Rohrabschnitt (28) zumindest annähernd zylindrisch gestaltet. Sie hat dort z.B. einen Innendurchmesser von 5,5 mm. Im Bereich des Gehäuseadapters (21) weitet sich die Zylinderinnenwandung (31) kegelstumpfmantelförmig auf. Der Kegelwinkel dieser Aufweitung (25) beträgt z.B. 50 Winkelgrade. Die Länge der Ausweitung (25) entspricht ca. einem Drittel der Länge des Gehäuseadapters (21).

Im Bereich des Bodenabschnitts (33) endet die Zylinderinnenwandung (31) in einem Zylinderboden (45), dessen Kegelwinkel z.B. 160 Winkelgrade beträgt. Zwischen dem Zylinderboden (45) und der im Ausblasrohr (54) angeordneten Austrittsdüse (60) befindet sich eine Ausblasbohrung (56), die zumindest im Bereich vor der Austrittsdüse (60) eine zylindrische Wandung hat. Der hintere, dem Zylinderboden (45) zugewandte Abschnitt der Bohrung (56) ist im Ausführungsbeispiel als Kegelstumpf ausgebildet. Er hat einen Kegelwinkel von z.B. 12 Winkelgraden. Er erstreckt sich beispielsweise im Bereich der Wandung der Bodenplatte (34).

Zwischen dem Ausblasrohr (54) und dem Ringsteg (51) ist nach Figur 1 im vorderen Bereich des Klebescheibenaufnahmeraums (53) eine Klebescheibe (110) angeordnet. Sie hat eine Materialstärke, die mindestens 0,4 mm größer ist als die Tiefe des Klebescheibenaufnahmeraums (53). Die Klebescheibe (110) hat eine zentrale Bohrung (122), deren Innendurchmesser z.B. um 0,5 - 1 mm kleiner ist als der Außendurchmesser des Ausblasrohres (54). Somit wird der vordere Bereich des Ausblasrohres (54) dicht und eng anliegend vom hinteren Bereich der Bohrung (122) umgeben. Der vordere, noch ungedehnte Bereich der Bohrung (122) erscheint somit in Figur 1 mit einen kleineren Durchmesser.

Die im Wesentlichen zylindrische Außenwandung der Klebescheibe (110) ist an der zylindrischen Innenwandung (52) des Ringstegs (51) geführt. Nach Figur 1 hat die Klebescheibe (110) im oberen Bereich ihrer Außenwandung einen radial, z.B. 0,5 mm, überstehenden Umlaufsteg (123), über den sie an der vorderen Innenkante (59) des Ringstegs (51) elastisch anliegt.

Zur Positionierung der Klebescheibe (110) am Ringsteg (51) des Bodenabschnitts (33), kann letzterer auch einen im vorderen Bereich des Ringstegs (51) angeformten, radial nach innen ragenden Steg aufweisen, der in eine entsprechende Ringnut der Klebescheibe (110) elastisch hineinragt.

Die z.B. aus Gummi oder einem anderen Elastomer gefertigte Klebescheibe (110) ist an ihren beiden plan ausgeführten Stirnseiten jeweils mit einer z.B. aus einem Haftkleber bestehenden Klebeschicht (121, 129) ausgestattet. Die restlichen Oberflächenbereiche haben eine gute Gleitfähigkeit, da die Klebescheibe (110) zumindest partiell mit Silikonöl behandelt oder teflonisiert wird.

In die Klebescheibe (110) kann eine Versteifungsscheibe (119) eingesetzt werden. Sie ist in Figur 2 gestrichelt dargestellt. Diese z.B. umspritzte oder einvulkanisierte Versteifungsscheibe (119) hat eine Wandstärke von z.B. 0,5 - 1 mm. Sie ist beispielsweise aus einem üblichen Eisen- oder Buntmetall gefertigt. Ihre Bohrung ist mindestens 1 mm größer als der Außendurchmesser des Ausblasrohres (54). Der Außendurchmesser der Versteifungsscheibe (119) ist z.B. 1 - 2 mm kleiner als der Außendurchmesser der Klebescheibe (110). Die hier in die Klebescheibe (110) integrierte Versteifungsscheibe (119) ist z.B. 0,5 bis 1 mm hinter der vorderen Klebeschicht 121) positioniert. Die Mittellinien der Klebescheibe (110) und der Versteifungsscheibe (119) sind deckungsgleich.

Ggf. hat die Klebescheibe (110) seitlich mindestens eine parallel zur Mittellinie (5) orientierte Kerbe, die es beim Einschieben der Klebescheibe (110) in den Klebescheibenaufnahmeraum (53) ermöglicht, die dort vorhandene Luft problemlos zu verdrängen. Die Luft kann auch über eine im Ringsteg (51) in der Nähe der Stirnfläche (46) des Bodenabschnitts (33) angeordnete Bohrung entweichen.

Das topfförmige Schutzgehäuse (150), ein Sterilverschluss, das beispielsweise aus Glas gefertigt ist, besteht hier aus einem rohrförmigen Mantel (151) und einem planen Boden (152). Die zylindrische, glatte Außenwandung (32) des Rohrabschnitts (28) und des Bodenabschnitts (33), mit der eingesetzten Klebescheibe (110) des Zylinders (20), wird hierbei von dem Schutzgehäuse (150) umgeben. Im Bereich des Rohrabschnitts (28) beträgt der Abstand zwischen dessen Außenwandung (32) und der Innenwandung (155) des Schutzgehäuses (150) z.B. 1,5 mm. Der axiale Abstand zwischen dem Boden (152) des Schutzgehäuses (150) und der Klebescheibe (110) beträgt nach Figur 1 z.B. 1 mm.

Am Zylinder (20) ist das Schutzgehäuse (150) an zwei Stellen lösbar fixiert. Die erste Stelle liegt am Übergang zwischen dem Rohrabschnitt (28) und dem Anschlagsteg (24) des Zylinders (20). Dort befindet sich, nach Figur 1, ein O-Ring (161), über den das Schutzgehäuse (150) gegenüber dem Zylinder (20) abgedichtet ist. Zugleich zentriert der O-Ring (161) das Schutzgehäuse (150) am Zylinder (20). Anstelle eines konventionellen O-Rings (161) kann auch ein Quadring, ein Profilring oder dergleichen verwendet werden.

Der Dichtring (161) wird bei der Montage zwischen dem Schutzgehäuse (150) und dem Zylinder (20) verklemmt, so dass er neben der Dichtfunktion auch problemlos eine Zentrier- und Haltefunktion übernehmen kann. Ggf. kann der Dichtring (161) auch durch einen abdichtenden, zähbleibenden Klebstoff ersetzt werden.

Die zweite Stelle zur Abstützung des Schutzgehäuses (150) am Zylinder (20) befindet sich mittig im Boden (152) des Schutzgehäuses (150). Dort ist eine zentrale Sacklochbohrung (156) angeordnet, die von einem am Boden (152) angeformten, nach innen vorstehenden Stützsteg (153) umgeben wird. Der ringförmige Stützsteg (153) liegt mit seiner z.B. halbtorusförmigen Stirnseite an der Klebescheibe (110) an.

In der Sacklochbohrung (156) steckt eingeklemmt oder eingeklebt ein abgestufter Gummistopfen (125). Letzterer sitzt abdichtend mit seinem hinteren Ende vorder Austrittsdüse (60) des Ausblasrohres (54). Sein vorderes, in der Sacklochbohrung (156) steckendes Ende weist einen Durchmesser auf, der z.B. 0,5 mm größer ist als der seines hinteren Endes. Der Gummistopfen (125) fixiert in radialer Richtung das vordere Ende des Schutzgehäuses (150) über die Klebescheibe (110), die sich am Ringsteg (51) des Zylinders (20) abstützt.

Im Schutzgehäuse (150) ist als feuchtigkeitsspeicherndes Material (171) z.B. ein rechteckiges Baumwollvlies (172) eingelegt. Das Baumwollvlies (172) liegt an der Innenwandung (155) des Schutzgehäuses (150) teil- oder vollflächig an. Vor dem Einlegen wird es z.B. mit destilliertem bzw. sterilem Wasser bis zur Sättigung angefeuchtet. Das Wasser kann zusätzlich mit entsprechenden Wirkstoffen gegen Verkeimung versetzt sein.

Nach Figur 1 ist der Zylinder (20) mit einer Injektionslösung (1) teilbefüllt. Der Flüssigkeitsspiegel (2) der Injektionslösung (1) befindet sich im Übergangsbereich zwischen dem Gehäuseadapter (21) und dem Rohrabschnitt (28). Auf dem Flüssigkeitsspiegel (2) ist blasenfrei und steril ein scheibenförmiger Dichtkörper (100) aufgesetzt, der unter einer radialen Klemmwirkung dichtend an der Zylinderinnenwandung (31) anliegt. Hinter dem Dichtkörper (100) ist ein topfförmiger Treibkörper (81) angeordnet. Der Treibkörper (81) liegt dabei am Dichtkörper (100) partiell an oder er hat einen Abstand von z.B. 0,2 bis 0,5 mm.

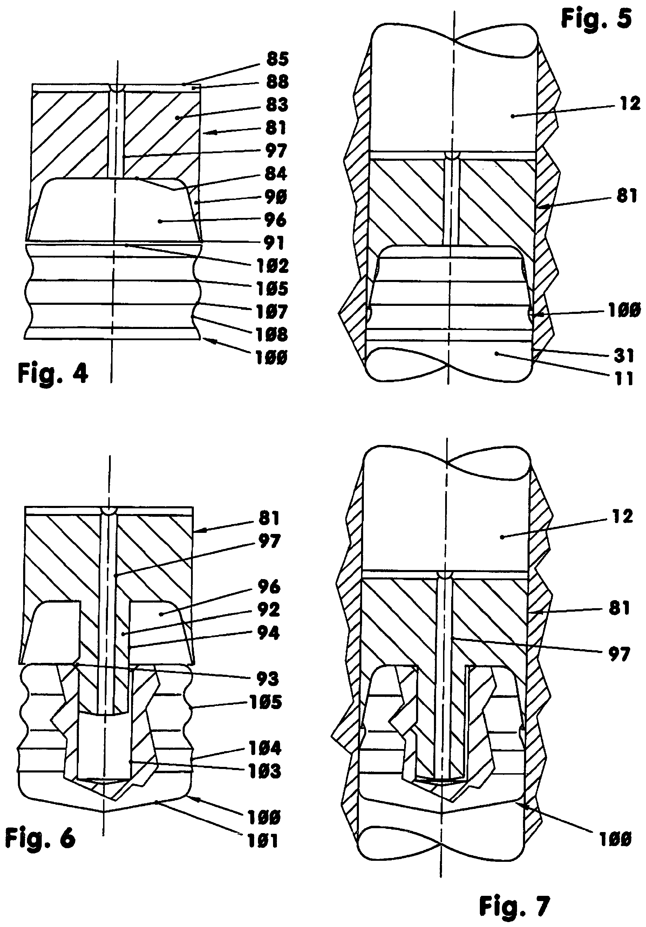

Der Dichtkörper (100) ist hier eine Scheibe, deren unverformter Durchmesser z.B. doppelt so groß ist wie seine Scheibendicke, vgl. Figur 4. Am Umfang weist diese Scheibe (100) z.B. ein Rillenprofil (107) auf, das z.B. zwei Rillen (108) hat. Das Rillenprofil (107) ist hier beispielsweise so gestaltet, dass der Dichtkörper (100) im Querschnitt beidseitig als Schnittprofil eine Wellenlinie mit zwei, die Rillen (108) formenden Wellentälern aufweist. Die Wellenlinie ist hierbei aus Kreisbögen zusammengesetzt.

Der Umfang der Scheibe (100) kann alternativ ein Rillenprofil für die endlos umlaufenden Stollen und Nuten haben, dessen Stollenquerschnitte z.B. durch Trapez,-Flach- und Sägengewinde bekannt sind. Bei den Stollenquerschnitten der Sägegewinde dürfen die Normalen der beiden Flanken eines Stollens Winkel bis zu 180 Winkelgraden einschließen.

Ggf. kann die radiale Außenwandung der Scheibe (100) auch eine glatte zylindrische Fläche sein.

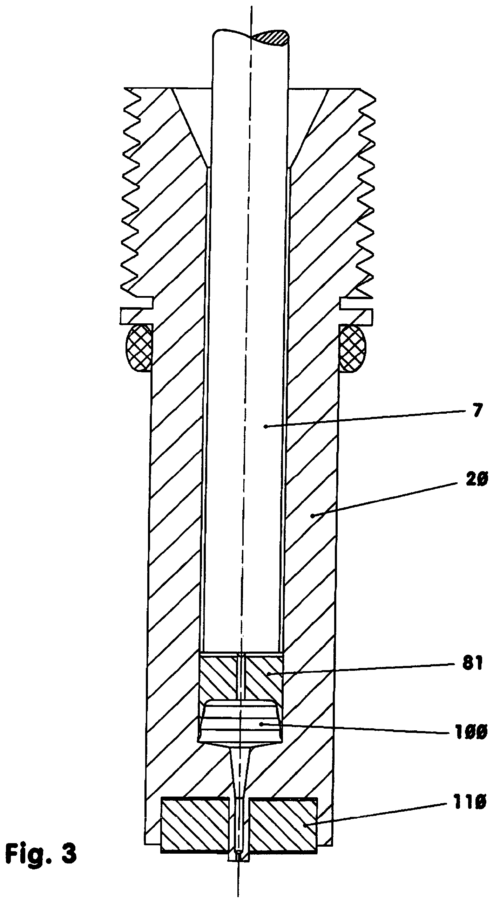

Da der Dichtkörper (100) ein elastisches, z.B. aus einem Elastomer gefertigtes Bauteil ist, sind die Wellenberge der gesetzten Dichtscheibe abgeplattet, vgl. Figuren 2, 3 und 5.

Der topfförmige Treibkörper (81), dessen Länge z.B. seinem Außendurchmesser entspricht, besteht aus einer scheibenförmigen Schlagplatte (83) und einer angeformten Schürze (90). Die Dicke der Schlagplatte (83) ist hierbei geringfügig größer als die Länge der Schürze (90), vgl. Figuren 4 und 5. Er kann u.a. aus Polytetrafluorethylen (PTFE), aus dem Perfluoralkoxy-Polymer (PFA), aus dem Fluorelastomer Perfluorethylenpropylen-Copolymer (FEP) oder aus dem Ethylen-Tetrafluorethylen (ETFE) hergestellt sein, wobei der erstgenannte Werkstoff den geringsten Reibkoeffizient hat.

Die Schlagplatte (83), auf die beim Auslösen des Injektors der Kolbenbetätigungsstempel (7) aufschlägt, hat mindestens eine z.B. zentrale Bohrung (97), die die vor und hinter dem Treibkörper (81) gelegenen Zylinderraumbereiche (11, 12) miteinander mit minimaler Drosselwirkung verbindet. Die Bohrung (97), die auch schräg zur Mittellinie (5) verlaufen kann, hat einen minimalen Durchmesser zwischen 1 und 2 mm. Sie endet nach den Ausführungsbeispielen an der hinteren Stirnseite (85) des Treibkörpers (81) z.B. in einem Kanalkreuz (88) aus zwei sich im Bereich der Bohrung (97) schneidenden Kanälen. Die Kanäle des Kanalkreuzes (88) haben jeweils einen halbkreisförmigen Querschnitt, wobei der Durchmesser der Querschnitte z.B. dem Durchmesser der Bohrung entspricht. Selbstverständlich können die Kanäle des Kanalkreuzes (88) auch dreieckige oder beliebig andere Querschnitte aufweisen.

An der vorderen Stirnseite (84) der Schlagplatte (83) schließt sich die als elastische Dichtlippe ausgebildete Schürze (90) an. Die Wandung der Schürze (90) verjüngt sich, ausgehend von der Stirnseite (84), hin zur vorderen, äußeren Dichtkante (91), die elastisch an der Zylinderinnenwandung (31) in jedem Betriebszustand des Injektors anliegt. Im eingebauten Zustand umschließen die Schürze (90) und die vordere Stirnseite (84) einen Eintauchhohlraum (96). Letzterer hat im Wesentlichen die Form eines Kegelstumpfes, dessen Kegelwinkel z.B. 20 Winkelgrade misst.

Selbstverständlich umfasst der von der Schlagplatte (83) und der Schürze (90) umschlossene Eintauchhohlraum (96) alle Formen von Vertiefungen, in die sich ein Dichtkörper (100) hineindrängen lässt. Der Eintauchholraum (96) kann so einen n-eckigen bis polygonalen Querschnitt haben. Mindestens ein Längsschnitt kann neben der beschrieben Trapezfläche eine Rechteckfläche, eine Dreiecksfläche, die Fläche eine Halbkreises, eines Kreisabschnitts, eines Kreisausschnitts oder dergleichen haben.

Die Figuren 6 und 7 zeigen einen Kolben (80) als Kombination aus dem Treibkörper (81) und dem Dichtkörper (100), bei der der Treibkörper (81) in der Sacklochbohrung (103) eines stopfenförmigen Dichtkörpers (100) über einen Zapfen (92) zentriert ist. Der Dichtkörper (100) hat hier - wie bei einer größeren Einmalspritze - einen explizit ausgebildeten Dichtsteg (104), der wenige zehntel Millimeter hinter einer kegeligen, vorderen Stirnseite (101) angeordnet ist. Nach hinten schließt sich an den Dichtsteg (104) ein Wellenprofil an, wie es zumindest annähernd aus Figur 4 bekannt ist. Die zentrale Sacklochbohrung (103) dieses elastischen Dichtkörpers (100) weist eine 0,5 mm breite 45°-Fase auf.

Der Zapfen (92) des Treibkörpers (81) hat nach Figur 6 auf seiner rechten Seite eine Abflachung (94), die sich über seine gesamte Länge erstreckt. Zudem hat er einen zumindest bereichsweise umlaufenden Anschlagsteg (93), über den sich der Treibkörper (81), bei noch nicht ausgelöstem Injektor, an der 45°-Fase der Sacklochbohrung (103) des Dichtkörpers (100) abstützt.

Die Sacklochbohrung (103) hat eine Tiefe, die zumindest annähernd so lang ist wie die Länge des Zapfens (92). In Figur 7 ist zu erkennen, dass beim Eindringen des Dichtkörpers (100) in den Eintauchhohlraum (96), die aus dem Eintauchhohlraum (96) zu verdrängende Luft entlang der Abflachung (94) in die Bohrung (97) einströmt, um von dort aus in den hinteren Zylinderraumbereich (12) zu gelangen.

Die Kombination (80) aus dem Treibkörper (81) und dem Dichtkörper (100) ermöglicht ein einfaches blasenfreies, steriles Befüllen und ein Verschließen der Zylinder-Kolben-Einheit (10) in Verbindung mit einem Ausstoßvorgang beim Auslösen des Injektors, der einem sehr hohen Verdichtungsstoß von bis zu 350 ^{∗} 10⁵ Pa standhält.

Zum blasenfreien Verschließen des mit der Injektionslösung (1) befüllten Zylinders (20) wird z.B. bei Umgebungsdruck die Dichtscheibe mit Hilfe eines Dreifingergreifers an ihrer Außenwandung (105) aufgenommen und durch die Finger des Dreifingergreifers in radialer Richtung soweit verdichtet, dass die Finger des Dreifingergreifers die Dichtscheibe (100) problemlos auf den Flüssigkeitsspiegel (2) im Zylinder (20) aufsetzen können. Die sich im Zylinder (20) spreizenden Finger des Dreifingergreifers werden zurückgezogen, während die hintere Stirnseite (102) der Dichtscheibe (100) in ihrer Position mittels eines Niederhalters gehalten wird.

Selbstverständlich lässt sich dieser Vorgang auch in einer Vakuumsetzmaschine realisieren.

Nach dem Setzen der Dichtscheibe (100) wird der Treibkörper (81) mit der Schürze (90) voraus in den Zylinder (20) hinter die Dichtscheibe (100) gesetzt. Der Treibkörper (81) haftet in dieser hinteren Position aufgrund der radialen Spannkraft der als elastische Dichtlippe ausgebildeten Schürze (90). Wie in den Figuren 4 und 6 ersichtlich, steht die elastische Schürze (90) - bei dem sich außerhalb des Zylinders (20) befindenden Treibkörper (81) - nach außen über die Außenkontur der Schlagplatte (83) über.

Wird der Injektor für die Injektion vorbereitet, wird das Schutzgehäuse (150) zusammen mit dem Baumwollvlies (172) nach vorn vom Zylinder (20) z.B. mittels Handkraft, abgezogen. Hierbei bleibt der Gummistopfen (125) am Schutzgehäuse (150) hängen, während der Dichtring (161) an der Außenwandung (32) des Zylinders (20) zurückbleibt.

Um die Applikation der Injektionslösung durchführen zu können, wird der Injektor mit der Klebescheibe (110) voraus auf die Hautoberfläche des Patienten aufgesetzt. Durch die Anpresskraft des Injektors wird die Klebescheibe (110) so belastet, dass sie - unter einem Überwinden der Sperrwirkung des Umlaufstegs (123) - entlang des Ausblasrohrs (54) in Richtung des Bodenabschnitts (33) verrutscht, um sich mit der Klebeschicht (129) der hinteren Stirnseite (115) an der Stirnfläche (46) des Bodenabschnitts (33) anzukleben. Die Klebescheibe (110) füllt nun den Klebescheibenaufnahmeraum (53) komplett aus. Bei diesem Vorgang verklebt zum einen die vordere Klebeschicht (121) der Klebescheibe (110) mit der Haut des Patienten und zum anderen tritt das Ausblasrohr (54) einige zehntel Millimeter aus der Klebescheibe (110) hervor.

Hierbei drückt die Stirnseite (58) des Ausblasrohres (54) eine Vertiefung in die Haut, um sie an der Applikationsstelle zusätzlich zu spannen. Gleichzeitig wird durch das Aufdrücken des Injektors auf die Haut, dieser ausgelöst. Der mittels einer mechanischen oder pneumatischen Feder vorgespannte Kolbenbetätigungsstempel (7) belastet schlagartig den Kolben (80), um mit der Injektionslösung (1) für die Einbringung in die Haut per Hochgeschwindigkeitsstrahl die gespannte Haut des Patienten zu durchschlagen.

Dabei trifft der Kolbenbetätigungsstempel (7) zunächst mit großer Wucht auf den Treibkörper (81), siehe Pfeilrichtung (3). Der Treibkörper (81) wird gegen die nahezu inkompressibel auf dem Flüssigkeitsspiegel (2) aufliegende Dichtscheibe (100) gepresst. Hierbei schiebt sich die Schürze (90) entlang der Zylinderinnenwandung (31) über die Außenwandung (105) der Dichtscheibe (100). Die Dichtscheibe (100) taucht in den Eintauchhohlraum (96) des Treibkörpers (81) ein, vgl. Figur 5 oder 7. Die dabei verdrängte Luft strömt über die Bohrung (97) und das Kanalkreuz (88) am Kolbenbetätigungsstempel (7) entlang in die äußere Injektorumgebung (9).

Nun bilden die Dichtscheibe (100) und der Treibkörper (81) einen quasistarren Verbund, den Kolben (80), der die Injektionslösung (1) vor sich herschiebt. Die Abdichtung gegenüber der Zylinderinnenwandung (31) übernimmt die Dichtkante (91) der Schürze (90), die durch die flüssigkeitsdruckbelastete Dichtscheibe (100) verstärkt angepresst wird. Da der Gleitreibungskoeffizient der Dichtkante (91), aufgrund des verwendeten Treibkörperwerkstoffes, kleiner ist als der Gleitreibungskoeffizient der Dichtscheibe (100), ergibt sich trotz hoher Dichtwirkung ein geringer Gleitreibungswiderstand.

### Bezugszeichenliste:

- 1: Injektionslösung
- 2: Flüssigkeitsspiegel
- 3: Pfeilrichtung bei Injektorauslösung
- 5: Mittellinie
- 7: Kolbenbetätigungsstempel
- 9: Umgebung

- 10: Zylinder-Kolben-Einheit
- 11: Zylinderraumbereich, vor (81)
- 12: Zylinderraumbereich, hinter (81)

- 20: Zylinder
- 21: Gehäuseadapter
- 22: Außengewinde
- 23: Schlitze
- 24: Anschlagsteg
- 25: Aufweitung, innen

- 28: Rohrabschnitt
- 29: Zylinderwandung
- 31: Zylinderinnenwandung, Innenwandung, radial
- 32: Außenwandung, radial
- 33: Bodenabschnitt
- 34: Bodenplatte

- 45: Zylinderboden, Innenseite des Zylinderbodens
- 46: Stirnfläche des Bodenabschnitts, vorn

- 51: Ringsteg
- 52: Innenwandung, zylindrisch
- 51: Ringsteg
- 52: Innenwandung, zylindrisch
- 53: Klebescheibenaufnahmeraum
- 54: Ausblasrohr, Kunststoff
- 56: Bohrung, Innenbohrung
- 58: Stirnseite, Stirnfläche
- 59: Kante, vordere
- 60: Düse, Austrittsdüse

- 80: Kolben, Kombination aus (81) und (100)
- 81: Treibkörper; Körper, topfförmig
- 83: Schlagplatte
- 84: Stirnseite, vorn
- 85: Stirnseite, hinten
- 88: Kanalkreuz

- 90: Schürze, elastisch; Dichtlippe
- 91: Kante, Dichtkante
- 92: Zapfen
- 93: Anschlagsteg
- 94: Abflachung, Kerbe
- 96: Eintauchhohlraum, Hohlraum
- 97: Ausnehmung, Bohrung, zentral

- 100: Dichtkörper, Dichtscheibe
- 101: Stirnseite, vorn
- 102: Stirnseite, hinten
- 103: Sacklochbohrung
- 104: Dichtsteg
- 105: Außenwandung, profiliert
- 107: Rillenprofil
- 108: Rille

- 110: Klebescheibe, Elastomerscheibe
- 115: Stirnseite, hinten
- 119: Versteifungsscheibe

- 121: Klebeschicht, vorn, Haftkleber, Klebebeschichtung
- 122: Bohrung, gestuft
- 123: Umlaufsteg
- 125: Gummistopfen
- 129: Klebeschicht, hinten, Haftkleber, Klebebeschichtung

- 50: Schutzgehäuse, Glas; Schale, außen; Sterilverschluss
- 151: Mantel, rohrförmig
- 152: Boden, plan
- 153: Stützsteg
- 155: Innenwandung
- 156: Sacklochbohrung
- 161: O-Ring

- 171: feuchtigkeitsspeicherndes Material Vlies, Baumwollvlies

## Patentansprüche

1. Zylinder-Kolben-Einheit (10) eines nadelfreien Injektors, mit einem, eine Injektionslösung aufnehmenden Zylinder (20) und einem Kolben (80),
- wobei der Kolben (80) aus einem Treibkörper (81) und aus mindestens einem Dichtkörper (100) besteht,
- wobei der mindestens eine Dichtkörper (100), der auf dem Flüssigkeitsspiegel (2) der Injektionslösung aufgesetzt ist, mindestens eine aus einem gummi- oder einem silikonartigen Werkstoff gefertigte Scheibe ist, die unter einer radialen Klemmwirkung an der Innenwandung (31) des Zylinders (20) anliegt,
- wobei der Treibkörper (81) ein topfförmiger Körper mit einer umlaufenden elastischen Schürze (90) ist,
- wobei zumindest ein Bereich der Schürze (90) unter einer radialen Klemmwirkung an der Innenwandung (31) des Zylinders (20) anliegt,
- wobei die vor und hinter dem Treibkörper (81) gelegenen Zylinderraumbereiche über mindestens eine Ausnehmung (97) des Treibkörpers (81) direkt oder über ein Ventil miteinander kommunizieren,
- wobei der Treibkörper (81) hinter dem mindestens einen Dichtkörper (100) im Zylinder (20) angeordnet ist und die Schürze (90) dem mindestens einen Dichtkörper (100) zugewandt ist und
- wobei für den bei Betätigung nach vorn verschiebbaren Treibkörper (81) der Dichtkörper (100) während der Vorwärtsfahrt nahezu komplett aufnehmbar ist, so dass der Verbund aus dem Treibkörper (81) und dem mindestens einen Dichtkörper (100) die Funktion eines Kolbens (80) hat.

2. Zylinder-Kolben-Einheit eines nadelfreien Injektors gemäß Anspruch 1,
**dadurch gekennzeichnet, dass** die radial orientierte Außenwandung (105) des Dichtkörpers (100) ein umlaufendes Rillenprofil (107) hat, dass mindestens eine Rille (108) umfasst.

3. Zylinder-Kolben-Einheit eines nadelfreien Injektors gemäß Anspruch 1,
**dadurch gekennzeichnet, dass** die Schürze des Treibkörpers (81) einen Hohlraum (96) umgibt, dessen Volumen minimal 50% und maximal 95% des Volumens des Dichtkörpers (100) entspricht.

4. Zylinder-Kolben-Einheit eines nadelfreien Injektors gemäß Anspruch 1,
**dadurch gekennzeichnet, dass** die Schürze (90) des Treibkörpers (81) eine elastisch nach radial außen federnde Dichtlippe ist, deren vordere, auf dem größten Durchmesser gelegene Kante (91) eine Dichtfunktion hat.

5. Zylinder-Kolben-Einheit eines nadelfreien Injektors gemäß Anspruch 1,
**dadurch gekennzeichnet, dass** der Treibkörper (81) aus einem fluorierten Kohlenwasserstoff hergestellt ist.

6. Zylinder-Kolben-Einheit eines nadelfreien Injektors gemäß Anspruch 5,
**dadurch gekennzeichnet, dass** der Werkstoff des Treibkörpers (81) aus einem selbstschmierenden Material gefertigt ist.

7. Zylinder-Kolben-Einheit eines nadelfreien Injektors gemäß Anspruch 1,
**dadurch gekennzeichnet, dass** die Ausnehmung (97) eine zentrale, zylindrische Bohrung des Treibkörpers (81) ist, die parallel zur Mittellinie (5) des Treibkörpers (81) orientiert ist.

8. Zylinder-Kolben-Einheit eines nadelfreien Injektors gemäß Anspruch 1,
**dadurch gekennzeichnet, dass** die Ausnehmung (97) an der hinteren Stirnseite (85) des Treibkörpers (81) in einem Kanalkreuz (88) endet.

9. Zylinder-Kolben-Einheit eines nadelfreien Injektors gemäß Anspruch 1,
**dadurch gekennzeichnet, dass** der Treibkörper (81) über einen Zapfen (92) in einer Sacklochbohrung (103) des Dichtkörpers (100) zentriert ist.

## Claims

1. A cylinder-piston unit (10) of a needle-free injector, comprising a cylinder (20) for accommodating an injection solution, and a piston (80),
- the piston (80) including a drive body (81) and at least one sealing body (100);
- the at least one sealing body (100), which is placed on the liquid level (2) of the injection solution, being at least one disk which is made of a rubber-like or a silicone-like material and, under a radial clamping action, bears against an inner wall (31) of the cylinder (20);
- the drive body (81) being a pot-shaped body having a peripheral elastic skirt (90);
- at least one area of the skirt (90), under a radial clamping action, bearing against the inner wall (31) of the cylinder (20);
- the cylinder chamber areas located in front of and behind the drive body (81) communicating with one another via at least one recess (97) of the drive body (81), either directly or by way of a valve;
- the drive body (81) being arranged behind the at least one sealing body (100) in the cylinder (20), and the skirt (90) facing the at least one sealing body (100); and
- the sealing body (100), during the forward travel, being substantially completely receivable by the drive body (81), which can be forwardly displaced upon actuation, so that the combination of the drive body (81) and of the at least one sealing body (100) has the function of a piston (80).

2. The cylinder-piston unit of a needle-free injector according to claim 1, **characterized in that** the radially oriented outer wall (105) of the sealing body (100) has a peripheral grooved profile (107), which comprises at least one groove (108).

3. The cylinder-piston unit of a needle-free injector according to claim 1, **characterized in that** the skirt (90) of the drive body (81) surrounds a cavity (96), having a volume that corresponds to at least 50% and no more than 95% of the volume of the sealing body (100).

4. The cylinder-piston unit of a needle-free injector according to claim 1, **characterized in that** the skirt (90) of the drive body (81) is a sealing lip which is elastically pre-loaded toward radially outside and the front edge (91) of which, located on the largest diameter, has a sealing function.

5. The cylinder-piston unit of a needle-free injector according to claim 1, **characterized in that** the drive body (81) is made of a fluorinated hydrocarbon.

6. The cylinder-piston unit of a needle-free injector according to claim 5, **characterized in that** the material of the drive body (81) is made of a self-lubricating material.

7. The cylinder-piston unit of a needle-free injector according to claim 1, **characterized in that** the recess (97) is a central, cylindrical borehole of the drive body (81) which is oriented parallel to the center line (5) of the drive body (81).

8. The cylinder-piston unit of a needle-free injector according to claim 1, **characterized in that** the recess (97) ends on the rear face (85) of the drive body (81) in a channel intersection (88).

9. The cylinder-piston unit of a needle-free injector according to claim 1, **characterized in that** the drive body (81) is centered in a blind borehole (103) of the sealing body (100) by way of a stub (92).

## Revendications

1. Unité cylindre-piston (10) d'un injecteur sans aiguille avec un cylindre (20) recevant une solution d'injection et un piston (80),
- où le piston (80) est constitué d'un corps d'entraînement (81) et d'au moins un corps d'étanchéité (100),
- où l'au moins un corps d'étanchéité (100), qui est posé sur le film de liquide (2) de la solution d'injection, est au moins un disque fabriqué à partir d'une matériau à base de caoutchouc ou de type silicone qui est adjacent à la paroi intérieure (31) du cylindre (20) moyennant un effet de serrage radial,
- où le corps d'entrainement (81) est un corps en forme de pot avec une collerette (90) élastique circonférentielle,
- où au moins une région de la collerette (90) est adjacente à la paroi intérieure (31) du cylindre (20) moyennant un effet de serrage radial,
- où les régions de la chambre cylindrique devant et derrière le corps d'entraînement (81) communiquent l'une avec l'autre de manière directe ou par le biais d'une soupape par au moins une cavité (97) du corps d'entraînement (81),
- où le corps d'entrainement (81) est disposé dans le cylindre (20) derrière l'au moins un corps d'étanchéité (100), et la collerette (90) est orientée vers l'au moins un corps d'étanchéité (100), et
- où, pour le corps d'entraînement (81) pouvant se déplacer vers l'avant lors de l'actionnement, le corps d'étanchéité (100) peut être pratiquement complètement parcouru pendant le trajet vers l'avant de sorte que l'ensemble à base du corps d'entraînement (81) et du corps d'étanchéité (100) a la fonction d'un piston (80).

2. Unité cylindre-piston d'un injecteur sans aiguille selon la revendication 1, **caractérisée en ce que** la paroi extérieure (105) orientée radialement du corps d'étanchéité (100) a un profilé rainuré (107) circonférentiel qui comprend au moins une rainure (108).

3. Unité cylindre-piston d'un injecteur sans aiguille selon la revendication 1, **caractérisée en ce que** la collerette du corps d'entraînement (81) entoure une cavité (96) dont le volume correspond au minimum à 50 % et au maximum à 95 % du volume du corps d'étanchéité (100).

4. Unité cylindre-piston d'un injecteur sans aiguille selon la revendication 1, **caractérisée en ce que** la collerette (90) du corps d'entraînement (81) est une lèvre d'étanchéité élastique faisant ressort radialement vers l'extérieur, dont l'arête (91) avant située sur le diamètre le plus grand a une fonction d'étanchéité.

5. Unité cylindre-piston d'un injecteur sans aiguille selon la revendication 1, **caractérisée en ce que** le corps d'entraînement (81) est fabriqué à base d'un hydrocarbure fluoré.

6. Unité cylindre-piston d'un injecteur sans aiguille selon la revendication 5, **caractérisée en ce que** la matière du corps d'entraînement (81) est fabriquée à base d'un matériau autolubrifiant.

7. Unité cylindre-piston d'un injecteur sans aiguille selon la revendication 1, **caractérisée en ce que** la cavité (97) est un alésage cylindrique central du corps d'entraînement (81) qui est orienté parallèlement à la ligne centrale (5) du corps d'entraînement (81).

8. Unité cylindre-piston d'un injecteur sans aiguille selon la revendication 1, **caractérisée en ce que** la cavité (97) se termine au niveau du côté frontal (85) arrière du corps d'entraînement (81) dans une croix de renfort (88).

9. Unité cylindre-piston d'un injecteur sans aiguille selon la revendication 1, **caractérisée en ce que** le corps d'entraînement (81) est centré dans un alésage borgne (103) du corps d'étanchéité (100) par le biais d'une bonde (92).
